# EUROPEAN PATENT SPECIFICATION

(11) **EP 1 542 672 B1**
(45) Date of publication and mention of the grant of the patent: **03.09.2008**
(21) Application number: 02807678.4
(22) Date of filing: 08.08.2002
(51) Int. Cl.: A61K 31/27

(54) **R-BAMBUTEROL, ITS PREPARATION AND THERAPEUTIC USES**
R-BAMBUTEROL, SEINE HERSTELLUNG UND THERAPEUTISCHE VERWENDUNGEN
R-BAMBUTEROL, SA PREPARATION ET SES UTILISATIONS THERAPEUTIQUES

(43) Date of publication of application: 22.06.2005
(73) Proprietor: Tan, Wen, DongGuan 523808, GuangZhou (CN)
(72) Inventor: Cheng, J.L., Worcester, MA 01605 (US); Tan, Wen, DongGuan 523808, GuangZhou (CN)
(74) Representative: Grünecker, Kinkeldey, Stockmair & Schwanhäusser Anwaltssozietät
(86) International application number: PCT/US2002/025225
(87) International publication number: WO 2004/014290

(56) References cited:
- WO-A-91/13615
- GB-A- 2 255 503
- US-A- 4 419 364
- CARLSSON, Y. ET AL: "Non-aqueous capillary electrophoretic separation of enantiomeric amines with (-)-2,3:4,6-di-O-isopropylidene-2-keto-L-g ulonic acid as chiral counter ion" JOURNAL OF CHROMATOGRAPHY, A , 922(1-2), 303-311 CODEN: JCRAEY; ISSN: 0021-9673, 2001, XP004255350

## Description

### FIELD OF THE INVENTION

The present invention relates to a compound having therapeutic activity, to a process for its preparation, and to the medicinal uses of the compound. The compound used in the invention has potent lipid-lowering effects in treatment of hyperlipidemia, particularly in hypertriglyceridemia.

### BACKGROUND OF THE INVENTION

The compounds of the general structural formula 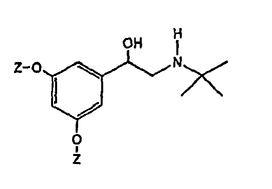 are examples of presently preferred long acting bronchodilator drugs on the market. The bronchodilator terbutaline (Z = H) is one such drug. Bambuterol (Z = C(O)NMe₂), the bis-dimethylcarbamate prodrug of terbutaline, has more potent bronchospasmolytic activity than the latter upon oral administration and exhibits a duration of activity of more than 12 hours. Bambuterol also exhibits a lower degree of undesired cardiovascular side effects. In addition, Bambuterol has lipid lowering effects in certain patients. (Bauer CA and Svensson LA, EP 0521967, 1990)

It is known that among many drugs having chiral centers, one enantiomer of a racemic pair is often more active than the other in treating a medical condition. For example, the levorotatory R-enantiomer of albuterol is approximately 80 times more potent as a β-2 receptor agonist than the dextrorotatory S-enantiomer (Hartley and Middlemis, J. Med. Chem., 14, 895-896,1971), and the administration of the pure R-enantiomer offers improved therapeutic activity and fewer side effects. The United States Food and Drug Administration has approved R-albuterol hydrochloride as a new drug for the treatment of asthma. Similar results in vivo were obtained with enantiomer of terbutaline.(Kallstrom et al., Chorality 1996,8,567)

The prodrug of terbutaline, bambuterol, has a chiral center, and it can exist as a racemic mixture or as pure enantiomeric forms (Torsten et al, U.S. Patent 4,419,364, 1983). Racemic bambuterol has been marketed and widely used clinically for several years. It is known that the S-isomers of β-2 agonists - including terbutaline - are more toxic or less potent than the R-isomers, properties which may be responsible for the side effects of racemic bambuterol in its clinical use. The present invention teaches the preparation of single enantiomers of bambuterol of high purity, and the superior therapeutic benefits of the single R-enantiomer of the drug over racemic bambuterol.

At least two synthetic approaches have been employed to control the stereochemistry of the chiral center of albuterol and related 2-phenylethanolamines. One approach utilized in the preparation of the enantiomerically pure R and S forms of albuterol is resolution of a racemic mixture of an intermediate in its synthesis or of the final compound itself (for review see Bakale et al., Clinical Reviews in Allergy and Immunology, Vol. 14, pp7-35, 1996). The second approach involves asymmetric synthesis, which is the de novo synthesis of a chiral substance from chiral precursors. GB 22 55 503 A discloses bronchodilator enantiomers.

### SUMMARY OF THE INVENTION

The present invention provides the subject matter of claims 1 to 7. The compound used in the invention has lipid-lowering activities; it can lower plasma lipids, particularly triglycerides, in hyperlipidemic conditions. The invention also relates to methods for preparation of the pure enantiomers of bambuterol and related 2-phenylethanolamines.

The invention defines the R enantiomer of bambuterol of the following structure: 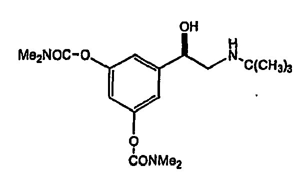 and therapeutically acceptable salts thereof. This compound is of use as a lipid-lowering drug in hyperlipidemias.

This invention also claims efficient and cost-effective synthetic methods for obtaining optically pure R-bambuterol and 2-phenylethanolamines and their synthetic intermediates, by resolution of racemic bambuterol and 2-phenylethanolamines by use of an enantioselective chemical reaction. The enantioselective synthetic method as defined in claim 7 comprises the steps of:
(a) asymmetrically reducing a suitably substituted and suitably protected α-bromoacetophenone to a chiral 2-bromo-1-phenylethanol; and
(b) displacing the bromo group by a suitably substituted and optionally protected primary amine to produce a chiral 2-phenylethanolamine.

All objects, features and advantages are of the present invention will be further detailed in the description that follows.

### DETAILED DESCRIPTION OF THE INVENTION

The invention relates to the R enantiomer of bambuterol, a compound of the following structure: 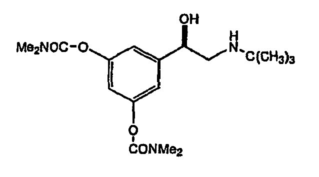 and therapeutically acceptable salts thereof.

The term "pure enantiomer" means a compound of the invention comprising at least 80% by weight of one of the two chiral forms and not more than 20% by weight of the other chiral form of the compound, or more preferably 98% by weight of one of the two chiral forms and not more than 2% by weight of the other chiral form of the compound, or even more preferably 99% by weight of one of the two chiral forms and not more than 1% by weight of the other chiral form of the compound

The method provided by the present invention relates a process for producing a single enantiomer of a 2-phenyl-2-ethanolamine, which is obtained by:
producing a suitably substituted 2-phenyl-2-ethanol bromide, a precursor to the 2-phenyl-2-ethanolamine, in high enantiomeric excess. The general steps of the methods are:
   (a) asymmetrically reducing a suitably substituted and suitably protected α-bromoacetophenone to a chiral 2-bromoethanol comprising a primary bromo group and a secondary hydroxyl group;
   (b) displacing the bromo by a suitably substituted and optionally protected primary amine to produce a chiral 2-phenyl-2-ethanolamine.

For the purposes of this invention, the term "suitable substituents" and equivalent terms mean substituents that when present, give rise to useful end products and intermediates thereof. In the case of chiral 2-phenyl-2-ethanolamines, preferred useful end products are β-adrenergic receptor agonists of the 2-phenyl-2-ethanolamine class including, among others, albuterol, formoterol, salmeterol, terbutaline, and bambuterol. Preferred useful intermediates of such compounds include the corresponding protected chiral 2-phenyl-2-ethanol bromide.

The term "suitable protecting group" means a group that protects an otherwise vulnerable chemical moiety of a particular compound in a specific reaction or reactions, and that can later be removed under conditions that do not destroy other functionalities that are present in the compound. Preferred suitable protecting groups for hydroxyl functionalities include ester, carbonate, carbamate, ketal, and related groups. Preferred suitable protecting groups for amine functionalities include amide, carbamate and related groups. A large number of suitable protecting groups and corresponding chemical cleavage reactions are described in "Protective Groups in Organic Chemistry", Theodore W. Greene (John Wiley & Sons, Inc., New York, 1991, ISBN 0-471-62301-6) ("Greene"). See also Kocienski, Philip J.; "Protecting Groups" (Georg Thieme Verlag Stuttgart, New York, 1994), which is incorporated by reference in its entirety herein. In particular Chapter 1, Protecting Groups: An Overview, pages 1-20, Chapter 2, Hydroxyl Protecting Groups, pages 21-94, Chapter 3, Diol Protecting Groups, pages 95-117, Chapter 4, Carboxyl Protecting Groups, pages 118-154, Chapter 5, Carbonyl Protecting Groups, pages 155-184.

The term "suitable leaving group" means a group that can be readily displaced from a reaction intermediate to produce a desired product without disturbing other functionalities present in the compound. Preferred suitable leaving groups for hydroxyl functionalities include tosylate, mesylate, trifluoroacetate and related groups. Bromine, chlorine and iodine are commonly used as leaving groups in organic synthesis.

In a preferred embodiment, the suitably substituted 2-phenyl-2-ethanol bromide is first obtained from its corresponding α-bromoacetophenone by methods well known to practitioners of the art, then the remaining steps of the process are performed. An example of this preferred embodiment is shown in Scheme 1.

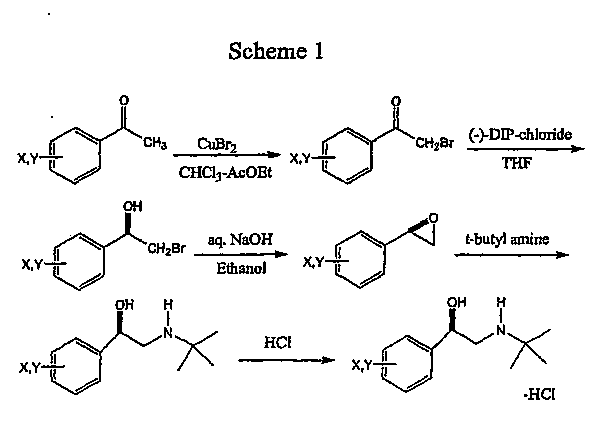

In this scheme, the key asymmetric reduction reagents are B-chlorodiisopinocampheylboranes (DIP-Chloride^{™}): (-)-DIP-chloride^{™} is used for preparation of "R" alcohol, and (+)-DIP-chloride^{™} for preparation of "S" alcohol. These reagents have been developed for the general preparation of chiral alcohols. (For reviews on the chemistry of DIP-chlorid^{™} see: Brown et al. Acc. Chem. Res. 1992, 25, 16; Brown and Ramachandran in Advances in Asymmetric Synthesis, Vol 1, Hassner, A., Ed., JAI Press: Greenwich, CT, 1994, pp-144-20; Ramachandran and Brown, in Reductions in Organic Synthesis, Chap. 5, Abdel-Magid, A., Ed., American Chemical Society: Washington, DC, 1996), and are available commercially or may be prepared in the laboratory by one skilled in the art.

The leaving group bromo is displaced by a primary amine resulting in the protected product. Depending on the requirements, protecting groups may be removed from substituents X, Y and/or Z to provide the final 2-phenyl-2-ethanolamine in high enantiomeric purity, typically ≥98%.

The groups X, Y and Z are substituents that impart value to the 2-phenyl-2-ethanolamines, such as activity as β-adrenergic agonists or growth promoters in livestock.. Examples of preferred substituents X and Y are hydroxyl, hydroxymethyl, amino, formamido, N, N-dimethylcarbamoyl and related groups. Examples of preferred substituent Z are tert-butyl, (CH₂)₆O(CH₂)₄-C₆H₅, CH(CH₃)-C₆H₄-4-OCH₃ and related groups.

Acid salts of the chiral 2-phenyl-2-ethanolamine products may also be prepared using the methods of this invention. Suitable salts include those derived from inorganic acids, such as sulfates and hydrochlorides, and those derived from organic acids, such as mesylates, fumarates , tartrates, citrates, maleates, succinates, and benzoates.

### Uses of R-bambuterol

In clinical use R-bambuterol, the compound used in the invention, will be administered orally, by injection or by inhalation, or may be absorbed via skin or rectum in the form of a pharmaceutical preparation comprising the compound of the invention as the active ingredient.

R-Bambuterol can be used:
(a) as a lipid lowering agent in hyperlipidemia or other related conditions
(b) to moblize excess fat tissue in animals or humans as a result of its plasma triglyceride lowering activity.

### EXAMPLES

### Preparation of R-bambuterol

R-bambuterol hydrochloride is prepared by the method of this invention as illustrated in Scheme 2. All reagents were available commercially. NMR spectra were recorded on a Bruker Avance instrument at 300MHz for ¹H. Chiral HPLC was done on a Waters instrument [column: Chiralcel OJ; mobile phase: 91(hexanes)/10(ethanol)/0.1(diethylamine); UV detection: 220 nm].

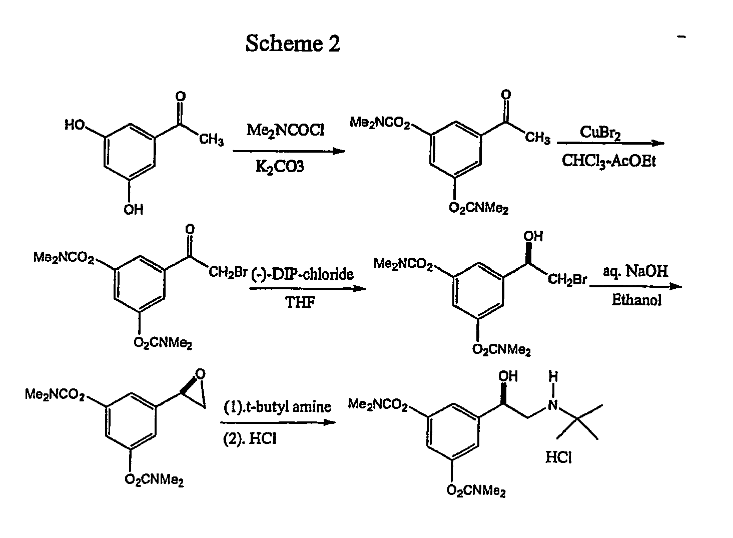

### Step 1. 3,5-di(N,N-dimethylcarbamyloxy)acetophenone

A mixture of 3,5-dihydroxyacetophenone (24 g, 0.16 mole), dimethylcarbamyl chloride (50 g, 0.46 mole), potassium carbonate 1.5 H₂O (41 g, 0.25 mole), anhydrous potassium carbonate (9.4 g, 0.07 mole) and pyridine (1 g) in ethyl acetate (150 mL) was stirred at 70°C for 2 hours. Water (120mL) was added to the mixture, and the resulting mixture was stirred at 70°C for 1.5 hours. After cooling to room temperature, the reaction mixture was separated, and the organic phase was washed with dilute sulfuric acid (2%), dried over MgSO₄, filtered, and the filtrate was concentrated to give the product: 40 g, yield 86%. ¹H NMR (CDCl₃) δ 2.58 (s, 3H, COCH₃), 2.90, 3.10 (s, 12H, 2xN(CH₃)₂), 7.20 (s, 1H, H4), 7.55 (s, 2H, H2,6).

### Step 2. 2'-Bromo-3,5-di(N,N-dimethylcarbamyloxy)acetophenone

A mixture of 3,5-di(N,N-dimethylcarbamyloxy)acetophenone (38 g, 0.13 mole), copper (II) bromide (57.7 g, 0.26 mole) in ethyl acetate (100 mL) and chloroform (100mL) was stirred at reflux for 5 hours. The mixture was filtered to remove the solid, and the filtrate was washed with water, dried over MgSO₄, and filtered. The filtrate was concentrated and the product crystallized: 44.1 g, yield 91 %. ¹H NMR (CDCl₃) δ 3.04, 3.12 (s, 12H, 2xN(CH₃)₂), 4.40 (s, 2H, CH₂), 7.25 (s, 1H, H4), 7.58 (s, 2H, H2,6) ppm.

### Step 3. (R)-1-Bromo-2- [3,5-bis(N,N-dimethylcarbamyloxy)phenyl)]-2-ethanol

A solution of 2'-bromo-3,5-di(N, N-dimethylcarbamyloxy)acetophenone (11 g, 30 mmole) in anhydrous tetrahydrofuran (100mL) was added to a solution of (-)-DIP-Chloride (10.6 g, 33 mmole) in anhydrous tetrahydrofuran (60mL) at -25°C under nitrogen. The resulting solution was stirred at -25°C for 60 hours, then warmed to 0°C, and diethanolamine (7 g, 66 mmole) was added dropwise. The mixture was warmed to room temperature and stirred for 2 hours, whereupon the boranes precipitated as a complex which was filtered and washed with pentane. The combined solvents were removed by distillation, and the residue was purified by silica gel column chromatography to give the product as an oil: 8.4 g, yield 75%. ¹H NMR (CDCl₃), consistent.

### Step 4. (S)-2-[3,5-di(N,N-dimethylcarbamyloxyl)phenyl]oxirane.

A 15% solution of NaOH in water (100mL) was added to a solution of (R)-1-bromo-2-[3,5-di(N, N-dimethylcarbamyloxy)phenyl)]-2-ethanol (7.0 g, 18.7 mmole) in ethanol (100mL). The mixture was stirred at room temperature for 2 hours. The reaction mixture was concentrated, treated with water, extracted with ethyl acetate. The organic layer was washed with brine and water, and dried over MgSO₄. The filtrate was evaporated and the residue dried under vacuum to give the product: 5.5 g, yield 100%. The product was used in the next step without further purification. 1H NMR (CDCl3) δ 2.56,3.15 (dd, 2H, CH2), 4.20 (dd, 1H, CH), 3.0, 3.10 (s, 12H, 2xN(CH3)2), 7.22 (s, 2H, H2,6), 7.56 (s, 1H, H4) ppm. Chiral HPLC, ee 98.7% (R, 99.35%, S, 0.65%).

### Step 5. R-Bambuterol hydrochloride.

A mixture of (S)-2-[3,5-di(N, N-dimethylcarbamyloxy)phenyl]oxirane (5.5 g, 18.7 mmole) in t-butylamine (80 mL) was stirred at reflux for 3 days. The mixture was concentrated to dryness, treated with water, and extracted with ethyl acetate. The organic layer was washed with water and dried over MgSO₄. The filtrate was concentrated and the residue treated with a solution of hydrogen chloride in diethyl ether to give R-bambuterol hydrochloride as white solid: 5.2 g, yield, 69%. ¹H and ¹³C NMR (D₂O) 1.30 (s, 9H, (CH3)3), 2.90, 3.06 (s, 12H, 2xN(CH3)2), 3.10, 3.25 (dd, 2H, H2',2"), 4.95 (dd, 1H, H1'), 6.88 (s,1H, H4), 7.06 (s, 2H, H2,6) ppm. Chiral HPLC, ee 99.4% (R, 99.7%, S, 0.3%).

### Pharmacologic tests of R-bambuterol hydrochloride

### A The lipid lowering effects of R-bambuterol

*Test method:* Male and 35 female mice, Kunming strain, 35 each, weight 18-22 grams, were used. Food and water were available ad libitum. The animals were randomized into 7 groups (n=10). Groups of animals were administered orally, via a stomach tube, vehicle (distilled water, two groups), R-bambuterol hydrochloride (10 mg/kg and 5 mg/kg), racemic bambuterol hydrochloride (10 mg/kg and 5 mg/kg) and simvastatin (10 mg/kg) once a day for four days. With the exception of one vehicle- treated group (as control), Tyloxapol (400 mg/kg, Sigma Chemical Co.) was injected into the peritoneal cavity of the mice of all groups immediately after the last treatment on the fourth day in order to induce hyperlipidemia. All the animals were fasted overnight prior to the injection of Tyloxapol. Blood samples were collected by cardiac puncture 24 hours after the injection of Tyloxapol. The blood samples were processed to plasma, and concentration of triglycerides (TG), cholesterol (CHO), high density lipoprotein (HDL), low density lipoprotein (LDL) and very low density lipoprotein (VLDL) were measured with an auto-blood analyzer (Olympic, Japan).

*Test result:* Administration of Tyloxapol to a vehicle-treated group resulted in significant increases in TG, CHO, LDL and VLDL, but not HDL, relative to the control, vehicle treated group without Tyloxapol (Table 6). However, there was significantly less or even no increases in TG, CHO, LDL and VLDL in the groups pre-treated with R-bambuterol, racemic bambuterol and simvastatin. These results are summarized in Table 6.

**Table 6 Lipid lowering effects of test compound in hyperlipidemia mice**

| | Lipid levels (mg/dl) | | | | |
|---|---|---|---|---|---|
| | TG | CHO | VLDL | LDL | HDL |
| Control* | 154 ± 25 | 104 ± 18 | 35 ± 6 | 20 ± 5 | 49 ± 8 |
| Tyloxapol# | 816 ± 115 | 240 ± 83 | 111 ± 54 | 54 ± 23 | 63 ± 20 |
| | | | | | |
| Tyl+R-bambuterol 5mg* | 323 ± 176 | 189 ± 83 | 50 ± 26 | 34 ± 15 | 42 ± 27 |
| Tyl+R-bambuterol 10mg* | 105 ± 18# | 119 ± 16 | 35 ± 7 | 29 ± 5 | 53.3 ± 6.7 |
| | | | | | |
| Tyl+bambuterol 10mg* | 148 ± 45 | 125.2±19.8 | 44.2±14.4 | 30 ± 6 | 50 ± 17 |
| Tyl+simvastatin 10mg@ | 311 ± 187* | 176 ± 64 | 85 ± 50 | 42 ± 11 | 49 ±16 |
| | | | | | |
| Tyl. Tyloxapol | | | | | |

| | | | | | |
|---|---|---|---|---|---|
| * Significant difference, in comparison with the values in the Tyloxapol group, in the levels of TG, CHO, VLDL and LDL, but not HDL In Tyl+simvastatin group only the value of TG was found significantly different from that in the Tyloxapol group. # Significant difference between the values in Tyl+R-bambuterol 10 mg and control groups @ Significant difference in comparison with the values in Tyl+R-bambuterol 10 mg group. No significant differences were found in HDL among all groups. | | | | | |

The results in Table 6 indicate that R-bambuterolo at both 5 mg/kg and 10 mg/kg significantly lowered triglycerides, cholesterol, VLDL and LDL in hyperlipidemic mice. At 10 mg/kg, R-bambuterol showed larger lowering effects than at 5 mg/kg, although only the differences in TG were significant (p<0.01). At 10 mg/kg, R-bambuterol lowered values in hyperlipidemic mice to the values similar to those in the control, non-hyperlipidemic group. R-bambuterol has significantly larger lowering effects on TG, CHO, VLDL and LDL in comparison with the same dose of simvastatin (10 mg/kg). The values in the hyperlipidemic groups treated with R-bambuterol (10mg/kg) or bambuterol (10 mg/kg) were similar to thosein control, non-hyperlipidemic mice. However, there is a further reduction in plasma triglycerides in hyperlipidemic mice treated with 10 mg/kg of R-bambuterol. The triglycerides in this group were significantly less than in the control, non-hyperlipidemic group. The same effect was not seen in the mice treated with either 10 mg/kg bambuterol or 10 mg/kg simvastatin.

### B. Cardiovascular effects of R-bambuterol in rats.

*Test method:* Rats (Sprague Dawley, 250-300g) were were randomized into two groups (n=6, equal sexes). After anesthesia, plastic tubing was introduced into a carotid artery of each animal and connected to pressure transducers to record mean artery pressure (MBP) and heart rate (HR). Another tube was introduced into the left ventricular chamber via the another carotid artery and connected to a pressure transducer. Left ventricular systolic pressure (LVSP) and the first order of differential of LVSP (dp/dt) as an index for inotropic effects were recorded. R-Bambuterol and racemic bambuterol hydrochloride were dissolved in saline and administrated intravenously at 30 mg/kg to each animal. The HR, MBP and Max dp/dt were measured before treatment (control) and after the treatments when the maximum response occurred, and 20 min after treatment as indication of recovery.

*Test result:* The results of these experiments are summarized in table 7.
R-Bambuterol showed some degree of chronotropic effects as indicated by increase in HR and a mild inotropic effects as indicated by the increase of Max dp/dt at the dose of 30mg/kg. These effects are not significant compared with racemic bambuterol hydrochloride of the same dose. However, the increase is significantly less in Max dp/dt in the rats treated with R-bambuterol (12.9% of control) than with racemic bambuterol (24.4% of control). This indicates that the test compound has less inotropic effects on the heart than racemic bambuterol at the dose given. During the recovery period (20min after treatment), the HR returned toward control (3.7 % of control) in animals treated with R-bambuterol, but the same value was significantly higher (9.5%) in animals treated with racemic bambuterol hydrochloride. This indicates that the chronotropic effect of R-bambuterol is significantly shorter in duration compared with racemic bambuterol.

**Table 7. Cardiovascular effects of R-bambuterol and bambuterol, 30 mg/kg, in rats.**

| | HR (Beat/min) | % of Control | MBP (Kpa) | % of control | Maxdp/dt (Kpa/sec) | % of control |
|---|---|---|---|---|---|---|
| R-Bambuterol: | | | | | | |
| Control | 402±38 | | 14.1±1.7 | | 297±84 | |
| Treatment Δ | 95±12 | 23.6% | 4.9± 0.8 | 34.8% | 38.4±3.5* | 12.9% |
| 20min afterΔ | 15±9* | 3.7% | 1.3±0.6 | 9.8% | 14.8±3 | 5.0% |
| | | | | | | |
| Rac-Bambuterol: | | | | | | |
| Control | 412±67 | | 13.3±1 | | 268±71 | |
| Treatment Δ | 85.8±5 | 20.6% | 4.2±0.5 | 31.6% | 65.4±9* | 24.4% |
| 20min afterΔ | 40±9* | 9.5% | 1.1±0.4 | 8.3% | 13±3.8 | 4.8% |

| | | | | | | |
|---|---|---|---|---|---|---|
| HR, heart rate. MBP, mean blood pressure. Max dp/dt, maximum value of first order differential of left ventricular systolic pressure. * significant difference in comparison with the same values in the bambuterol group respectively (p<0.01) | | | | | | |

Comments on the results of pharmacological tests of R-bambuterol.

### R-Bambuterol:

1. has potent lipid-lowering effects in hyperlipidemic animals, an effect which can restore the plasma triglycerides, cholesterol, VLDL and LDL the level of normal control animals. R-Bambuterol has significantly stronger effects on lowering plasma triglycerides than racemic bambuterol and simvastatin in hyperlipidemic animals.
2. has less chronotropic and inotropic side effects than the same dose of racemic bambuterol in rats.

## Claims

1. A composition comprising substantially optically pure R-Bambuterol or its pharmaceutically acceptable salt, wherein the R-Bambuterol is the R-enantiomer of bambuterol of the following structure, 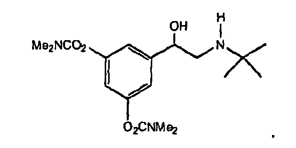 , comprising at least 80% by weight of the R-enantiomer and not more than 20% by weight of the S-enantiomer based on a total weight of the bambuterol, for use in the treatment of hyperlipidemia as hypertriglyceridemia a obesity or other metabolic disorders of lipid or fatty acids.

2. The composition of claim 1, wherein the composition is for oral, intravenous, subcutaneous or skin or rectum absorptive use.

3. The composition according to claim 1 comprising at least 98.5% by weight of the R-enantiomer and not more than 1.5% by weight of the S-enantiomer based on a total weight of the bambuterol.

4. Use of a composition comprising substantially optically pure R-Bambuterol or its pharmaceutically acceptable salt, wherein the R-Bambuterol is the R-enantiomer of bambuterol of the following structure, 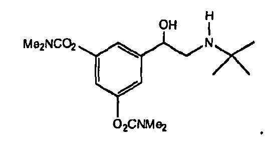 comprising at least 80% by weight of the R-enantiomer and not more than 20% by weight of the S-enantiomer based on a total weight of the bambuterol, for the preparation of a medicament for the treatment of hyperlipidemia or hypertriglyceridemia, obesity or other metabolic disorders of lipid or fatty acids.

5. The use of claim 4, wherein the medicament is for oral, intravenous, subcutaneous or skin or rectum absorptive use.

6. The use according to claim 4 comprising at least 98.5% by weight of the R-enantiomer and not more than 1.5% by weight of the S-enantiomer based on a total weight of the bambuterol.

7. A process for preparing a R-bambuterol composition wherein the composition comprises substantially optically pure R-Bambuterol or its pharmaceutically acceptable salt, wherein the R-Bambuterol is the R-enantiomer of bambuterol of the following structure, 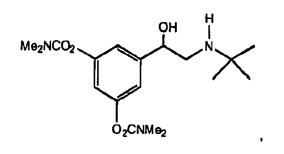 comprising at least 80% by weight of the R-enantiomer and not more than 20% by weight of the S-enantiomer based on a total weight of the bambuterol, comprising the steps of:
(a) asymmetrically reducing, using (-)-DIP-chloride (for R enantiomer) a suitably substituted and suitably protected α-bromoacetophenone compound to a chiral phenyl-bromoethanol comprising a primary bromo group and a secondary hydroxyl group;
(b) displacing the bromo group by a suitably substituted and optionally protected primary amine to produce a chiral phenylethanolamine, wherein the chiral phenylethanolamine is R-bambuterol or a pharmaceutically acceptable salt thereof.

## Patentansprüche

1. Zusammensetzung, umfassend im wesentlichen optisch reines R-Bambuterol oder ein pharmazeutisch akzeptables Salz davon, wobei das R-Bambuterol das R-Enantiomer von Bambuterol mit der folgenden Struktur ist, 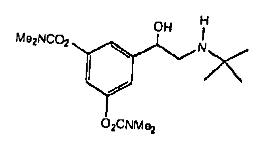 umfassend mindestens 80 Gew.-% des R-Enantiomeren und nicht mehr als 20 Gew.-% des S-Enantiomeren, basierend auf dem Gesamtgewicht an Bambuterol, zur Verwendung bei der Behandlung von Hyperlipidemia oder Hypertriglyceridemia oder Fettleibigkeit oder andere metabolische Störungen von Lipiden oder Fettsäuren.

2. Zusammensetzung nach Anspruch 1, wobei die Zusammensetzung für orale, intravenöse, subkutane oder transdermale oder rektale Absorption verwendet wird.

3. Zusammensetzung nach Anspruch 1, umfassend mindestens 98,5 Gew.-% des R-Enantiomeren und nicht mehr als 1,5 Gew.-% des S-Enantiomeren, basierend auf dem Gesamtgewicht an Bambuterol.

4. Verwendung einer Zusammensetzung, umfassend im wesentlichen optisch reines R-Bambuterol oder ein pharmazeutisch akzeptables Salz davon, wobei das R-Bambuterol das R-Enantiomer von Bambuterol mit der folgenden Struktur ist, 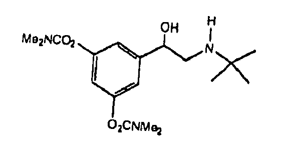 umfassend mindestens 80 Gew.-% des R-Enantiomeren und nicht mehr als 20 Gew.-% des S-Enantiomeren, basierend auf dem Gesamtgewicht an Bambuterol, zur Herstellung eines Medikaments zur Behandlung von Hyperlipidemia oder Hypertriglyceridemia oder Fettleibigkeit oder anderen metabolischen Strörungen von Lipiden oder Fettsäuren.

5. Verwendung nach Anspruch 4, wobei die Zusammensetzung für orale, intravenöse, subkutane oder transdermale oder rektale Absorption verwendet wird.

6. Verwendung nach Anspruch 4, umfassend mindestens 98,5 Gew.-% des R-Enantiomeren und nicht mehr als 1,5 Gew.-% des S-Enantiomeren, basierend auf dem Gesamtgewicht an Bambuterol.

7. Verfahren zur Herstellung von einer R-Bambuterolzusammensetzung, wobei die Zusammensetzung im wesentlichen optisch reines R-Bambuterol oder ein pharmazeutisch akzeptables Salz davon umfasst, wobei das R-Bambuterol das R-Enantiomer von Bambuterol mit der folgenden Struktur ist, 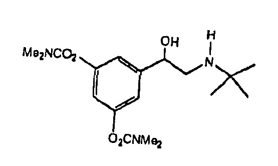 umfassend mindestens 80 Gew.-% des R-Enantiomeren und nicht mehr als 20 Gew.-% des S-Enantiomeren, basierend auf dem Gesamtgewicht an Bambuterol, umfassend die Stufen:
(a) Asymmetrische Reduktion, unter Verwendung von (-)-DIP-Chlorid (für das R-Enantiomer), einer geeignet substituierten oder geeignet geschützten α-Bromoacetophenonverbindung zu einem chiralen Phenyl-bromoethanol, umfassend eine primäre Bromogruppe und eine sekundäre Hydroxylgruppe;
(b) Ersetzen der Bromogruppe durch eine geeignet substituierte und geeignet geschützte primäre Aminfunktion um chirales Phenylethanolamin zu ergeben, wobei das chirale Phenylethanolamin R-Bambuterol oder ein pharmazeutisch akzeptables Salz davon ist.

## Revendications

1. Composition comprenant du R-bambutérol sensiblement optiquement pur ou son sel pharmaceutiquement acceptable, dans laquelle le R-bambutérol est l'énantiomère R du bambutérol de structure suivante, 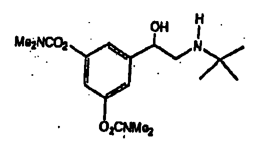 qui comprend au moins 80% en poids de l'énantiomère R et pas plus de 20% en poids de l'énantiomère S par rapport au poids total du bambutérol, pour utilisation dans le traitement de l'hyperlipidémie ou de l'hypertriglycéridémie, de l'obésité ou d'autres troubles métaboliques des lipides ou des acides gras.

2. Composition selon la revendication 1, laquelle composition est destinée à un usage oral, intraveineux, sous-cutanée ou par absorption par la peau ou le rectum.

3. Composition selon la revendication 1, comprenant au moins 98, 5% en poids de l' énantiomère R et pas plus de 1,5% en poids de l'énantiomère S par rapport au poids total du bambutérol.

4. Utilisation d'une composition comprenant du R-bambutérol sensiblement optiquement pur ou son sel pharmaceutiquement acceptable, dans laquelle le R-bambutérol est l'énantiomère R du bambutérol de structure suivante, 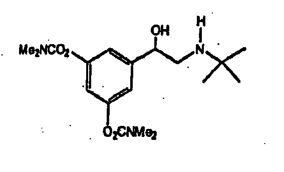 qui comprend au moins 80% en poids de l'énantiomère R et pas plus de 20% en poids de l'énantiomère S par rapport au poids total du bambutérol, pour la préparation d'un médicament pour le traitement de l'hyperlipidémie ou de l'hypertriglycéridémie, de l'obésité ou d'autres troubles métaboliques des lipides ou des acides gras.

5. Utilisation selon la revendication 4, dans laquelle le médicament est destiné à un usage oral, intraveineux, sous-cutanée ou par absorption par la peau ou le rectum.

6. Utilisation selon la revendication 4, qui comprend au moins 98,5% en poids de l'énantiomère R et pas plus de 1,5% en poids de l'énantiomère S par rapport au poids total du bambutérol.

7. Procédé pour la préparation d'une composition de R-bambutérol, dans lequel la composition comprend du R-bambutérol sensiblement optiquement pur ou son sel pharmaceutiquement acceptable, le R-bambutérol étant l'énantiomère R du bambutérol de structure suivante, 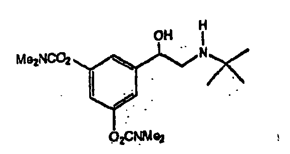 composition qui comprend au moins 80% en poids de l'énantiomère R et pas plus de 20% en poids de l'énantiomère S par rapport au poids total du bambutérol, lequel procédé comprend les étapes de :
(a) réduction asymétrique, en utilisant le (-)-DIP-chloride (pour l'énantiomère R), d'un composé α-bromoacétophénone substitué de manière appropriée et protégé de manière appropriée en un phényl-bromoéthanol chiral comprenant un groupe bromo primaire et un groupe hydroxyle secondaire;
(b) déplacement du groupe bromo par une amine primaire substituée de manière appropriée et éventuellement protégée de manière appropriée pour produire une phényléthanolamine chirale, ladite phényléthanolamine chirale étant le R-bambutérol ou un sel pharmaceutiquement acceptable de celui-ci.
